# EUROPEAN PATENT SPECIFICATION

(11) **EP 4 460 268 B1**
(45) Date of publication and mention of the grant of the patent: **22.04.2026**
(21) Application number: 23708998.2
(22) Date of filing: 06.02.2023
(51) Int. Cl.: A61F 5/00

(54) **IMPLANTABLE DEVICE FOR PYLORIC OCCLUSION AND/OR DUODENAL EXCLUSION**
IMPLANTIERBARE VORRICHTUNG FÜR PYLORUSOKKLUSION UND/ODER DUODENALEXKLUSION
DISPOSITIF IMPLANTABLE D'OCCLUSION PYLORIQUE ET/OU D'EXCLUSION DUODÉNALE

(30) Priority: 07.02.2022 US 202263307333 P
(43) Date of publication of application: 13.11.2024
(73) Proprietor: Boston Scientific Scimed, Inc., Maple Grove, MN 55311 (US)
(72) Inventor: CURRAN, Darren, Gerard, Galway, H91H2RV (IE); COLLINS, David, Galway (IE); O'DRISCOLL, John, Galway (IE); BOLGER, Brian, Pierce, Kilkenny (IE); LENEHAN, Rebecca, Shammer Kilkelly, F35DK20 (IE); POWER, Francis, Galway (IE)
(74) Representative: Vossius & Partner Patentanwälte Rechtsanwälte mbB
(86) International application number: PCT/US2023/012406
(87) International publication number: WO 2023/150344

(56) References cited:
- US-B2- 7 803 195

## Description

### FIELD

The present disclosure relates generally to the field of implantable devices for reducing and/or occluding passage of material through a body passage or lumen. More particularly, the present disclosure relates to devices for reducing and/or occluding passage of material through a body passage or lumen in the gastrointestinal tract, such as the pylorus.

### BACKGROUND

Various medical devices, systems, and methods exists for occluding or obstructing a body passage. For instance, in the gastrointestinal (GI) discipline, various devices have been designed for occluding or obstructing or reducing flow through a lumen in the GI system, such as the pyloric valve (pylorus). Treatment methods for various medical conditions, such as obesity, diabetes, or duodenal ulcers, involve bypassing the duodenum or restricting flow of materials through the duodenum by occluding the pylorus with an occlusion device to inhibit or block passage of materials (fluid, chyme, etc.) from the stomach through the pylorus and into the duodenum. Various challenges to preventing migration of a deployed occlusion device are presented by the natural movements of the body (e.g., the GI system) as well as the constant flow of materials against the occlusion device. Peristaltic movement of the pylorus to pass materials therethrough (e.g., distally into the small intestine), generally less frequent reverse peristalsis through the pylorus (proximally into the stomach), as well as the natural tendency of the pylorus to eject materials therein present particular challenges for placement and retention of pyloric occlusion devices. Moreover, in addition to resisting migration, it may also be desirable for the occlusion device to be removable without damaging the tissue at the deployment site. Accordingly, there is still a need for improvements to occlusion devices, such pyloric plugs, to resist migration while providing the desired amount of occlusion, and, optionally, allow reversibility of occlusion.

US 7,803,195 B2 discloses a method and apparatus for treating obesity which includes an artificial fistula created between gastrointestinal organs such as between the stomach and the colon. The method includes selecting an implant comprising a passageway having an internal lumen with an inlet end and an outlet end. The passageway is positioned passing through a first wall of first gastrointestinal organ (for example, passing through the wall of the stomach) and a second wall of a second gastrointestinal organ (for example, passing through the wall of the large intestine) with the inlet end disposed within an interior of the first gastrointestinal organ and with the outlet disposed within an interior of the second gastrointestinal organ.

### SUMMARY

In accordance with various principles of the present disclosure, an implantable device includes a tissue-contacting component configured to resist tissue ingrowth and an anti-migration component separately formed from the tissue-contacting component and configured to hold the tissue-contacting component in place with respect to tissue at a deployment site.

In some embodiments, the tissue-contacting component is coated with a material resistant to tissue ingrowth.

In some embodiments, at least one of the tissue-contacting component or the anti-migration component is expandable from a compact delivery configuration to an expanded deployment configuration. In some embodiments, the tissue-contacting component has a lumen defined therethrough; and the anti-migration component is expandable within the lumen defined through the tissue-contacting component to exert an anti-migration force on the tissue-contacting component to hold the tissue-contacting component in place with respect to tissue at the deployment site. In some embodiments, the tissue-contacting component is an expandable stent; and the anti-migration component is an expandable balloon. In some embodiments, at least one of the tissue-contacting component or the anti-migration component includes at least one retention member extending radially-outwardly therefrom. In some embodiments, the at least one retention member extends radially outwardly from an end of said anti-migration component.

In some embodiments, the tissue-contacting component has a proximal end configured to be seated in a stomach, a distal end configured to be seated in a duodenum, and a saddle region extending between the proximal end and the distal end and configured to be seated in a pylorus; and the anti-migration component has a proximal end with a proximal retention member extending radially therefrom and configured to be seated in the stomach, a distal end with a distal retention member extending radially therefrom and configured to be seated in the duodenum, and a saddle region extending between the proximal end and the distal end and configured to extend through the saddle region of the tissue-contacting component. In some embodiments, the distal retention member is curved to correspond to the curve of the duodenum in which the distal retention member is seated. In some embodiments, at least one of the tissue-contacting component or the anti-migration component includes at least one retention member extending radially-outwardly therefrom. In some embodiments, the at least one retention member extends radially outwardly from an end of the anti-migration component.

In accordance with various principles of the present disclosure an occlusion device configured to occlude a body passage includes a first component defining a lumen therethrough and configured to extend through the body passage; and a second component formed separately from the first component and extending through the lumen of the first component. In some embodiments, the first component is coated to prevent tissue ingrowth therein, and the second component exerts a radially outward anti-migration force on the first component to retain the first component in place with respect to the body passage.

In some embodiments, the second component occludes flow of materials through the lumen defined through the first component.

In some embodiments, at least one of the first component or the second component includes at least one retention member extending radially-outwardly therefrom. In some embodiments, the at least one retention member extends radially outwardly from an end of the second component and has a diameter greater than the diameter of the lumen through the first component and is configured to resist migration through the first component. In some embodiments, the diameter of the at least one retention member extending radially outwardly from an end of the second component is greater than the diameter of the body passage through which the first component extends and is configured to resist migration of the first component and the second component through the body passage.

In some embodiments, the first component is an expandable stent, and the second component is an expandable balloon.

In accordance with various principles of the present disclosure, a method of occluding flow of materials through a body passage includes delivering a first component of an occlusion device through the body passage, the first component having a lumen extending therethrough and covered with a coating prevent tissue ingrowth therein; delivering a second component of an occlusion device through the lumen of the first component, and expanding the second component to hold the first component in place within the body passage.

In some embodiments, the method includes extending a saddle region of the first component through the pylorus, and expanding the second component within the lumen defined through the first component to retain the first component within the pylorus. In some embodiments, the method includes expanding at least one end of the second component to have a diameter greater than the diameter of the lumen defined through the first component and to resist migration of the second component with respect to the first component. In some embodiments, the method includes expanding the at least one end of the second component to have a diameter greater than the diameter of the body passage and to resist migration of the second component with respect to the body passage.

In some embodiments, the second component is delivered after delivering the first component.

These and other features and advantages of the present disclosure, will be readily apparent from the following detailed description, the scope of the claimed invention being set out in the appended claims. While the following disclosure is presented in terms of aspects or embodiments, it should be appreciated that individual aspects can be claimed separately or in combination with aspects and features of that embodiment or any other embodiment.

### BRIEF DESCRIPTION OF THE DRAWINGS

Non-limiting embodiments of the present disclosure are described by way of example with reference to the accompanying drawings, which are schematic and not intended to be drawn to scale. The accompanying drawings are provided for purposes of illustration only, and the dimensions, positions, order, and relative sizes reflected in the figures in the drawings may vary. For example, devices may be enlarged so that detail is discernable, but is intended to be scaled down in relation to, *e.g.,* fit within a working channel of a delivery catheter or endoscope. In the figures, identical or nearly identical or equivalent elements are typically represented by the same reference characters, and similar elements are typically designated with similar reference numbers differing in increments of 100, with redundant description omitted. For purposes of clarity and simplicity, not every element is labeled in every figure, nor is every element of each embodiment shown where illustration is not necessary to allow those of ordinary skill in the art to understand the disclosure.

The detailed description will be better understood in conjunction with the accompanying drawings, wherein like reference characters represent like elements, as follows:
**FIG. 1** illustrates a schematic representation of a portion of a gastrointestinal system with an occlusion device, such as formed in accordance with various principles of the present disclosure, positioned across a pylorus.
**FIG. 2** illustrates an example of an embodiment of an occlusion device formed in accordance with various principles of the present disclosure.
**FIG. 3** illustrates another example of an embodiment of an occlusion device formed in accordance with various principles of the present disclosure.
**FIG. 4** illustrates another example of an embodiment of an occlusion device formed in accordance with various principles of the present disclosure.
**FIG. 5** illustrates another example of an embodiment of an occlusion device formed in accordance with various principles of the present disclosure.
**FIG. 6** illustrates another example of an embodiment of an occlusion device formed in accordance with various principles of the present disclosure.
**FIG. 7** illustrates a schematic representation of a portion of a gastrointestinal system with an occlusion device such as illustrated in **FIG. 6** positioned therein.
**FIG. 8** illustrates a schematic representation of a portion of a gastrointestinal system with the anti-migration component of an occlusion device such as illustrated in **FIG. 6** (and without the tissue-engaging component) positioned therein.

### DETAILED DESCRIPTION

The following detailed description should be read with reference to the drawings, which depict illustrative embodiments. It is to be understood that the disclosure is not limited to the particular embodiments described, as such may vary. All apparatuses and systems and methods discussed herein are examples of apparatuses and/or systems and/or methods implemented in accordance with one or more principles of this disclosure. Each example of an embodiment is provided by way of explanation and is not the only way to implement these principles but are merely examples. Thus, references to elements or structures or features in the drawings must be appreciated as references to examples of embodiments of the disclosure, and should not be understood as limiting the disclosure to the specific elements, structures, or features illustrated. Other examples of manners of implementing the disclosed principles will occur to a person of ordinary skill in the art upon reading this disclosure. In fact, it will be apparent to those skilled in the art that various modifications and variations can be made in the present disclosure without departing from the scope or spirit of the present subject matter. For instance, features illustrated or described as part of one embodiment can be used with another embodiment to yield a still further embodiment. Thus, it is intended that the present subject matter covers such modifications and variations as come within the scope of the appended claims and their equivalents.

It will be appreciated that the present disclosure is set forth in various levels of detail in this application. In certain instances, details that are not necessary for one of ordinary skill in the art to understand the disclosure, or that render other details difficult to perceive may have been omitted. The terminology used herein is for the purpose of describing particular embodiments only, and is not intended to be limiting beyond the scope of the appended claims. Unless defined otherwise, technical terms used herein are to be understood as commonly understood by one of ordinary skill in the art to which the disclosure belongs. All of the devices and/or methods disclosed and claimed herein can be made and executed without undue experimentation in light of the present disclosure.

As used herein, "proximal" refers to the direction or location closest to the user (medical professional or clinician or technician or operator or physician, etc., such terms being used interchangeably herein without intent to limit, and including automated controller systems or otherwise), etc., such as when using a device (e.g., introducing the device into a patient, or during implantation, positioning, or delivery), and/or closest to a delivery device, and "distal" refers to the direction or location furthest from the user, such as when using the device (e.g., introducing the device into a patient, or during implantation, positioning, or delivery), and/or closest to a delivery device. "Longitudinal" means extending along the longer or larger dimension of an element. "Central" means at least generally bisecting a center point and/or generally equidistant from a periphery or boundary, and a "central axis" means, with respect to an opening, a line that at least generally bisects a center point of the opening, extending longitudinally along the length of the opening when the opening comprises, for example, a tubular element, a channel, a cavity, or a bore.

Various implantable devices may be configured to be securely implanted within a body to resist migration from the implant site, yet may also be configured to be removable if desired or medically indicated. An example of such implantable devices includes implantable devices configured for insertion into a body passage to occlude flow of materials through the body passage. It will be appreciated that the terms body passage and lumen may be used interchangeably herein without intent to limit, the broad principles of the present disclosure being applicable to various shapes and sizes of body passages / lumens. Such devices may be referenced as occlusion, exclusion, closure, obstruction, etc., devices, such terms (and other grammatical forms thereof) being used interchangeably herein without intent to limit. The site or location at which the device is inserted, positioned, deployed, implanted, extended, placed, etc., (such terms and other grammatical forms thereof being used interchangeably herein without intent to limit) within a human body may be referenced interchangeably herein as a treatment site or a deployment site or implant site without intent to limit. It will be appreciated that the deployment site generally may be understood to be the intended treatment site or position of the device once deployed and in use. It will further be appreciated that reference may be made to placement of a device at, through, across, in, into, etc., a deployment site, such terms being used interchangeably herein without intent to limit.

An example of an occlusion device is a pyloric occlusion device. Duodenal or pyloric exclusion as an endoscopic alternative to gastric bypass is still a relatively new concept, and there are still various challenges being discovered. Two such challenges are removability of the device (such as for replacement, or upon completion of treatment) and anti-migration (such as which may otherwise be caused by natural motility, peristaltic motions, pressure of materials being blocked by the device, etc.). An occlusion device formed in accordance with various principles of the present disclosure has one or more components sized, shaped, configured, dimensioned, etc., to address at least these challenges.

To address removability, at least a tissue-engaging component of an implantable occlusion device which is in contact with tissue at the treatment site is formed and configured in accordance with various principles of the present disclosure to be readily removable (e.g., without affecting, or damaging, the tissue at the treatment site). For instance, such tissue-engaging component of the occlusion device may be configured to resist tissue ingrowth therein. For instance, the tissue-engaging component of the occlusion device may be coated or covered with a material known in the art to inhibit or prevent tissue ingrowth into the device, such as along regions in contact with tissue at the treatment site. Various known sheaths or coatings, such as biocompatible, polymeric, optionally lubricious, coatings, or heretofore known coatings, may be applied to selected regions of the tissue-engaging component of the occlusion device to contribute to the mechanics of the device, such as to impart structural stability, and/or to inhibit tissue ingrowth. In some embodiments, the material of the sheath or coating may be selected to increase the pull out force of the occlusion device, such as by virtue of the viscosity of the material, and/or the ultimate durometer of the material upon curing. In one aspect, the tissue-engaging component of the occlusion device is in the form of an expandable stent which may have interstitial spaces, and the coating covers the stent to inhibit or prevent ingrowth of tissue over or into the stent, such as into the interstitial spaces of the stent. It will be appreciated that other forms and configurations of implantable occlusion devices and/or components thereof which resist tissue ingrowth therein are within the scope and spirit of the present disclosure. It will be appreciated that such solution to removability obviates the need for tissue ingrowth promotion treatments (e.g., pretreatments), such as ablation.

As may be appreciated by those of ordinary skill in the art, a challenge presented by facilitating removability of an implantable device such as an occlusion device is the risk of migration. Current data indicates that to prevent an implantable device from migrating, tissue ingrowth is important. For instance, use of bare, uncoated stents, and ablating the treatment site (e.g., pyloric area) before device deployment has been found to be very effective in reducing migration of the implanted device. An implantable device which is readily removable, such as by provision of a coating partially or fully covering the device to inhibit or prevent tissue ingrowth, thus generally is susceptible to migration without further modifications to the device.

In accordance with various principles of the present disclosure, to address anti-migration of a removable implantable device, an anti-migration component is operatively associated with the tissue-engaging component to hold or wedge the tissue-engaging component in place with respect to the deployment site. For instance, the anti-migration component operatively engages the tissue-engaging component to hold the tissue-engaging component against tissue at the deployment site. In some embodiments, the anti-migration component is separate from (formed separately from) the tissue-engaging component. In some embodiments, the anti-migration component is deployed separately from (e.g., after) the tissue-engaging component is deployed. In some embodiments, the anti-migration component is expandable with respect to the tissue-engaging component. In some embodiments, the anti-migration component is an inflatable balloon, such as an inflatable molded balloon. The balloon may be formed of a compliant or non-compliant material. In some embodiments, the balloon may be molded into a configuration further promoting occlusion of a body passage and/or anti-migration in addition to wedging the tissue-engaging component in place. Any desired fluid, such as air or saline, may be used to inflate the balloon. Additionally or alternatively, hydrogel beads or other materials may be used to inflate the balloon to increase the pullout force of the balloon. In some embodiments, the anti-migration component may be readily removable (e.g., by deflating, such as puncturing or otherwise releasing fluid from within an anti-migration component in the form of a balloon) so that a separate tissue-engaging component is not needed.

In some embodiments, an implantable device is configured to be deployed in a body passage to occlude the body passage, and the tissue-engaging component may have a lumen therethrough. The anti-migration component may be inserted into the lumen of the tissue-engaging component. In some embodiments, the anti-migration component is expanded within the lumen of the tissue-engaging component to hold the tissue-engaging component in place against the walls of the body passage in which the implantable device is positioned. For instance, in some embodiments, expansion of the anti-migration component creates a radial expansion force which wedges the tissue-engaging component in place with respect to the body passage. The anti-migration component, as positioned within the lumen of the tissue-engaging component, may also serve to close off / occlude the body passage by occluding the tissue-engaging component positioned therein.

The tissue-engaging component may include at least one retention member extending radially-outwardly therefrom and configured to retain the tissue-engaging component with respect to the deployment site. For instance, a retention member may extend radially outwardly from (to be understood herein to encompass at or adjacent) one or both ends of the tissue-engaging component with larger diameters than an intermediate saddle region of the tissue-engaging component. The retention members may be sized, shaped, configured, and/or dimensioned to be seated (e.g., anchored) against tissue at the deployment site of the occlusion device. For instance, if the saddle region of the occlusion device is positioned through a body passage, then the retention members may be radially expanded members configured to be seated against tissue surrounding the body passage in which the implantable device is positioned to hold the tissue-engaging component in place with respect to the body passage (e.g., to prevent migration of the tissue-engaging configuration from its position within the body passage).

Additionally or alternatively, the anti-migration component may include at least one retention member, such as extending radially outwardly from (to be understood herein to encompass at or adjacent) one or both ends of the anti-migration component. The retention members of the anti-migration component may be sized, shaped, configured, and/or dimensioned to resist migration of the anti-migration component with respect to the tissue-engaging component and/or the body passage in which the occlusion device is positioned. For instance, the retention members of an anti-migration component extending through a lumen defined through an intermediate saddle region of a tissue-engaging component may have diameters larger than the diameter of the lumen of the tissue-engaging component to resist migration therethrough. The retention members of the anti-migration component may be sized, shaped, configured, and/or dimensioned to be seated (e.g., anchored) against tissue surrounding the body passage in which the implantable device is positioned to hold the anti-migration component in place. Optionally, the retention members of the anti-migration component are seated with respect to tissue surrounding the body passage to hold the retention members of the tissue-engaging component in place as well (e.g., with the retention members of the tissue-engaging component between the retention members of the anti-migration component and the tissue surrounding the body passage). Such retention members may serve to increase the pullout force of the tissue-engaging component and/or the anti-migration component to prevent migration of the implantable device.

Various embodiments of an occlusion device and method formed in accordance with various principles of the present disclosure will now be described with reference to examples illustrated in the accompanying drawings. Reference in this specification to "one embodiment," "an embodiment," "some embodiments", "other embodiments", etc. indicates that one or more particular features, structures, and/or characteristics in accordance with principles of the present disclosure may be included in connection with the embodiment. However, such references do not necessarily mean that all embodiments include the particular features, structures, and/or characteristics, or that an embodiment includes all features, structures, and/or characteristics. Some embodiments may include one or more such features, structures, and/or characteristics, in various combinations thereof. Moreover, references to "one embodiment," "an embodiment," "some embodiments", "other embodiments", etc. in various places in the specification are not necessarily all referring to the same embodiment, nor are separate or alternative embodiments necessarily mutually exclusive of other embodiments. When particular features, structures, and/or characteristics are described in connection with one embodiment, it should be understood that such features, structures, and/or characteristics may also be used in connection with other embodiments whether or not explicitly described, unless clearly stated to the contrary. It should further be understood that such features, structures, and/or characteristics may be used or present singly or in various combinations with one another to create alternative embodiments which are considered part of the present disclosure, as it would be too cumbersome to describe all of the numerous possible combinations and subcombinations of features, structures, and/or characteristics. Moreover, various features, structures, and/or characteristics are described which may be exhibited by some embodiments and not by others. Similarly, various features, structures, and/or characteristics or requirements are described which may be features, structures, and/or characteristics or requirements for some embodiments but may not be features, structures, and/or characteristics or requirements for other embodiments. Therefore, the present disclosure is not limited to only the embodiments specifically described herein, and the examples of embodiments disclosed herein are not intended as limiting the broader aspects of the present disclosure.

In the accompanying drawings, it will be appreciated that common features are identified by common reference elements and, for the sake of brevity and convenience, and without intent to limit, the descriptions of the common features are generally not repeated. For purposes of clarity, not all components having the same reference number are numbered. It will be appreciated that, in the following description, elements or components similar among the various illustrated embodiments are generally designated with the same reference numbers increased by a multiple of 100 and redundant description is generally omitted for the sake of brevity. Moreover, certain features in one embodiment may be used across different embodiments and are not necessarily individually labeled when appearing in different embodiments.

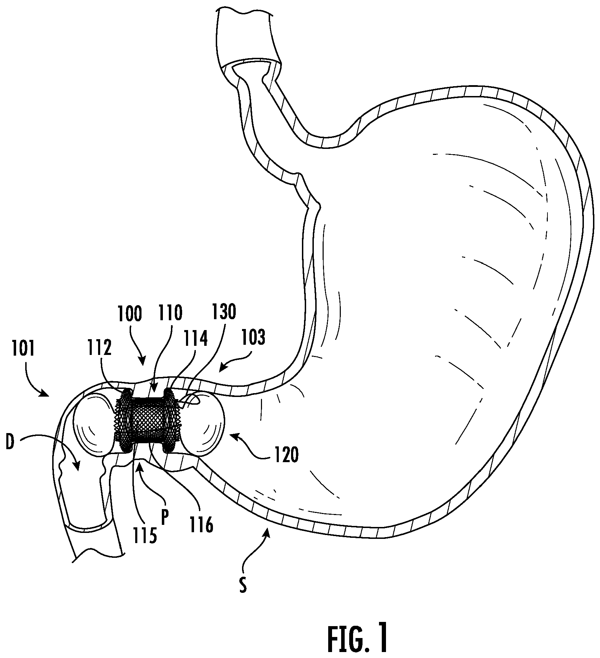

Turning now to **FIG. 1****,** an example of an embodiment of an implantable device, such as an occlusion device **100,** formed in accordance with various principles of the present disclosure to allow removability as well as to resist migration (i.e., to have anti-migration features) is illustrated positioned across a pylorus **P** with a distal end **101** positioned in a duodenum **D** and a proximal end **103** positioned in a stomach **S** (with a saddle region **116,** between the distal end **101** and proximal end **103,** positioned within the pylorus **P).** It will be appreciated that such placement is only one example, and various principles of the present disclosure may be applied to other types of implantable devices positioned in other parts of the body with respect to different anatomical structures.

In accordance with various principles of the present disclosure, the occlusion device **100** includes a tissue-engaging component **110** and an anti-migration component **120.** The tissue-engaging component **110** is a removable component and generally in contact with tissue at the treatment site (in this case, the pylorus **P),** yet treated or otherwise configured to be removable, such as to inhibit tissue growth with respect thereto. The anti-migration component **120** is operatively associated with the tissue-engaging component **110** to hold the tissue-engaging component **110** in place with respect to the treatment site. For instance, in the illustrated embodiment, the tissue-engaging component **110** is configured to be placed in a body passage and has a lumen **115** therethrough (e.g., defined by the saddle region **116),** and the anti-migration component **120** is positioned within lumen of the tissue-engaging component **110** and wedges the tissue-engaging component **110** in place between the anti-migration component **120** and the treatment site. The tissue-engaging component **110** typically is deployed before the anti-migration component **120** is deployed (e.g., sequentially), or both components **110, 120** may be deployed together (e.g., simultaneously, such as with the anti-migration component **120** positioned within the lumen **115** defined by the saddle region **116** of the tissue-engaging component **110).** It will be appreciated that other configurations and embodiments are within the scope and spirit of the present disclosure.

The tissue-engaging component **110, 210, 310, 410, 510, 610** of an occlusion device **100, 200, 300, 400, 500, 600** formed in accordance with various principles of the present disclosure may be in the form of a stent, as illustrated in **FIGS. 1-7****.** The stent may be formed of a biocompatible metal, a polymer, or a combination thereof. The stent may be configured to shift between a delivery configuration (e.g., collapsed, constrained, unexpanded, etc.) in which the stent is configured to facilitate delivery through a body passage (e.g., through a delivery catheter extended transluminally to the deployment site), and a deployment configuration (e.g., expanded) in which the stent is configured to facilitate deployment at a deployment site, such as by contacting the walls of the body passage in which the stent is placed. The stent may be self-expanding, or may be expanded with the assistance of another device (e.g., an expandable balloon). Examples of stents include, without limitation, stents having one or more strut members combined and/or arranged to form a rigid and/or semi-rigid stent structure. In some embodiments, the occlusion device is formed of one or more wires or filaments which are braided, wrapped, intertwined, interwoven, woven, knitted, looped (e.g., bobbinet-style), knotted, or the like to form a scaffold configuration. Alternatively, the stent may be a monolithic structure formed from a cylindrical tubular member, such as a single, cylindrical laser-cut tubular member, in which the remaining portions of the tubular member form the strut members. The strut members of a self-expanding stent may be formed of heat-formable material or a shape-memory material, such as Nitinol or Elgiloy, so that the stent returns to a pre-shaped expanded configuration from a collapsed configuration. Openings may be defined between adjacent strut members or filaments of the stent. It will be appreciated that the term openings is used for the sake of convenience, and may be used interchangeably herein with such terms as spaces or interstices or interstitial spaces or the like without intent to limit.

In accordance with various principles of the present disclosure, the tissue-engaging component **110, 210, 310, 410, 510, 610** of an occlusion device **100, 200, 300, 400, 500, 600** is configured to facilitate removability from the treatment site. For instance, the tissue-engaging component **110, 210, 310, 410, 510, 610** may be covered with a cover or coating configured to inhibit or prevent ingrowth of tissue (e.g., into the openings in the walls thereof), which may facilitate removal of the occlusion device **100, 200, 300, 400, 500, 600** if desired or medically indicated. For instance, a coating may be applied to at least a portion of the tissue-engaging component **110, 210, 310, 410, 510, 610** to inhibit or prevent tissue ingrowth with respect to the tissue-engaging component **110, 210, 310, 410, 510, 610** (such as tissue ingrowth into the structure of the tissue-engaging component **110, 210, 310, 410, 510, 610,** such as into interstices or openings therein or in a wall thereof). The coating may be formed of a suitable biocompatible, optionally lubricious, material known or heretofore known in the art, such as a polymeric material, such as silicone, polyurethane, polyvinylidene difluoride (PVDF), polytetrafluorethylene (PTFE), expanded polytetrafluoroethylene (ePTFE), polyester, polypropylene, polyethylene, polynaphthalene, Chronoflex^{®}, etc., and/or copolymers thereof, and/or combinations thereof.

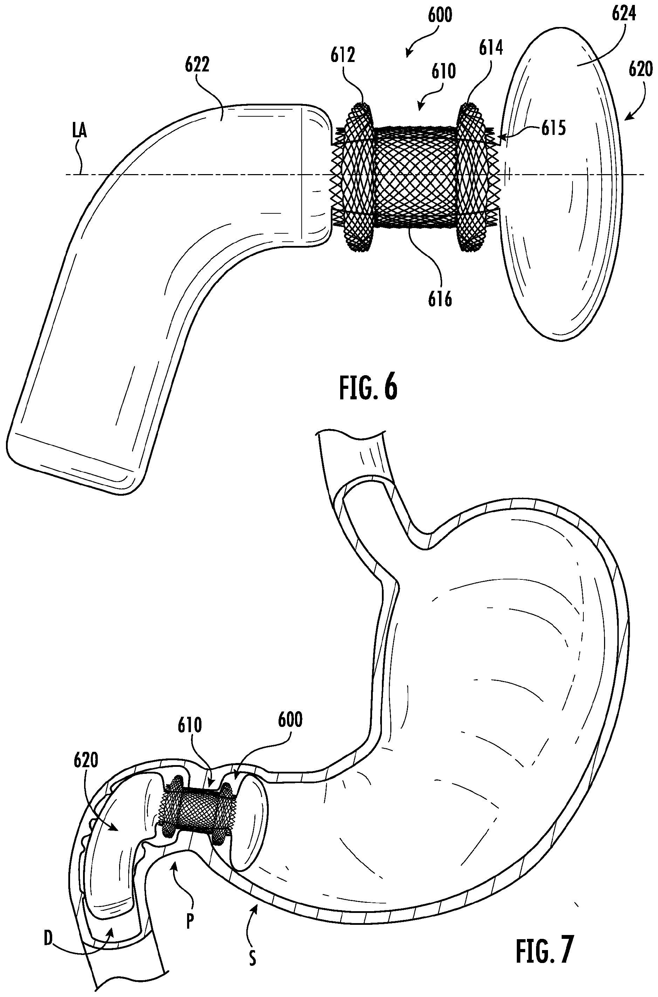

In accordance with various principles of the present disclosure, to inhibit undesired migration, the tissue-engaging component **110, 210, 310, 410, 510, 610** of an occlusion device **100, 200, 300, 400, 500, 600** configured to extend through a body passage may be sized, shaped, configured, and/or dimensioned to resist migrating out of such body passage. For instance, the tissue-engaging component **110, 210, 310, 410, 510, 610** may include a distal retention member **112, 212, 312, 412, 512, 612** configured to be seated against tissue surrounding a distal opening of the body passage (in the examples illustrated in **FIG. 1** and **FIG. 7****,** a duodenum **D),** and a proximal retention member **114, 214, 314, 414, 514, 614** configured to be seated against tissue surrounding a proximal opening of the body passage (in the example illustrated in **FIG. 1** and **FIG. 7****,** a stomach **S),** with a saddle region **116, 216, 316, 416, 516, 616** extending therebetween and through the body passage (in the example illustrated in **FIG. 1** and **FIG. 7****,** a pylorus **P).** In some embodiments, one or both of the retention members **112, 212, 312, 412, 512, 612, 114, 214, 314, 414, 514, 614** are double-wall retention members, the double walls of each retention member being adjacent each other (as referenced herein, adjacent and in contact or spaced apart from each other).

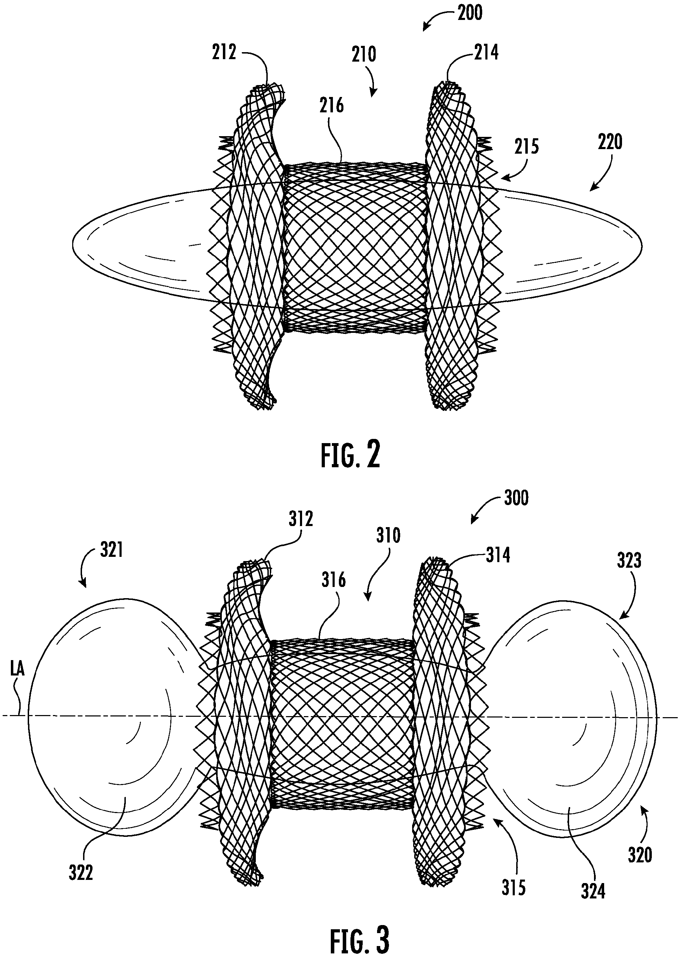

The retention members **112, 212, 312, 412, 512, 612, 114, 214, 314, 414, 514, 614** may be sized, shaped, configured, and/or dimensioned to inhibit or prevent unintentional migration of the tissue-engaging component **110, 210, 310, 410, 510, 610** from the body passage (e.g., distally into the duodenum **D,** or proximally into the stomach **S).** For instance, in the example of an embodiment of an occlusion device **200** illustrated in **FIG. 2****,** at least the distal retention member **212** of the example of an embodiment of a tissue-engaging component **210** has a concave surface (e.g., inner wall of a double-wall retention member **212)** facing toward the body passage. Such contour resists proximal migration in the direction of the body passage, increasing the pullout force of the distal retention member **212.** The distal retention member **312** of the example of an embodiment of a tissue-engaging component **310** of the example of an embodiment of an occlusion device **300** of **FIG. 3** is illustrated with a similar concave surface facing toward the body passage. It will be appreciated that the proximal retention member **214, 314** of the respective tissue-engaging components **210, 310** of the occlusion devices **200, 300 of** **FIG. 2** and **FIG. 3** may have a similar concave surface facing the body passage to resist distal migration in the direction of the body passage. Various modifications to sizes, shapes, dimensions, and configurations of one or both walls of a distal retention member **112, 212, 312, 412, 512, 612,** and/or a proximal retention member **114, 214, 314, 414, 514, 614** to enhance antimigration characteristics of the tissue-engaging component **110, 210, 310, 410, 510, 610** and/or to increase pullout force of the retention member **112, 212, 312, 412, 512, 612, 114, 214, 314, 414, 514, 614** are within the scope and spirit of the present disclosure, including, without limitation, as shown and described in U.S. Patent Application Publication 2022/0346997, filed on April 28, 2022, and published on November 3, 2022.

As noted above, to further inhibit undesired migration of an occlusion device **100, 200, 300, 400, 500, 600** formed in accordance with various principles of the present disclosure, an anti-migration component **120, 220, 320, 420, 520, 620** is operatively associated with the tissue-engaging component **110, 210, 310, 410, 510, 610** to hold the tissue-engaging component **110, 210, 310, 410, 510, 610** in place with respect to the deployment site. In the examples illustrated in **FIGS. 1-7****,** the anti-migration component **120. 220, 320, 420, 520, 620** is configured to retain the tissue-engaging component **110, 210, 310, 410, 510, 610** within a body passage (e.g., a duodenum **D,** such as illustrated **FIG. 1** and **FIG. 7****).** More particularly, the saddle region **116, 216, 316, 416, 516, 616** of the respective tissue-engaging component **110, 210, 310, 410, 510, 610** defines a lumen **115, 215, 315, 415, 515, 615** therethrough, and through which a respective anti-migration component **120, 220, 320, 420, 520, 620** may be extended. Like the tissue-engaging component **110, 210, 310, 410, 510, 610,** the associated respective anti-migration component **120, 220, 320, 420, 520, 620** may be configured to shift between a delivery configuration (e.g., collapsed, constrained, unexpanded, etc.) in which the anti-migration component **120, 220, 320, 420, 520, 620** is configured to facilitate delivery through a body passage (e.g., through a delivery catheter extended transluminally to the deployment site), and a deployment configuration in which the anti-migration component **120, 220, 320, 420, 520, 620** is expanded to engage the deployment site. In some embodiments, an expandable anti-migration component **120, 220, 320, 420, 520, 620** may be used to expand the tissue-engaging component **110, 210, 310, 410, 510, 610** if the tissue-engaging component **110** is not self-expanding.

The anti-migration component **120, 220, 320, 420, 520, 620** is configured to be positioned within the lumen **115, 215, 315, 415, 515, 615** of the associated respective tissue-engaging component **110, 210, 310, 410, 510, 610** to exert a force on the tissue-engaging component **110, 210, 310, 410, 510, 610** serving as an antimigration force to inhibit or prevent the tissue-engaging component **110, 210, 310, 410, 510, 610** from migrating from the deployment site (e.g., out of the body passage in which it has been deployed). Such force may be referenced interchangeably herein as an anti-migration or holding or retaining or retention force without intent to limit. In an embodiment in which the anti-migration component **120, 220, 320, 420, 520, 620** is expandable within the lumen **115, 215, 315, 415, 515*,* 615** of the associated respective tissue-engaging component **110, 210, 310, 410, 510, 610,** the antimigration force may be controlled and selected by the medical professional by controlling or selecting the amount by which the anti-migration component **120, 220, 320, 420, 520, 620** is expanded to achieve the desired degree of retention force without exerting an excess amount of force which may have undesired consequences. In some embodiments, the anti-migration component **120, 220, 320, 420, 520, 620** may be configured so that it exerts only up to a preselected maximum expansion force. In some embodiments, the anti-migration component **120, 220, 320, 420, 520, 620** may be an inflatable balloon, formed of a compliant or non-compliant material. A medical professional may control or select the volume or pressure of fluid transmitted into the inflatable anti-migration component **120, 220, 320, 420, 520, 620** to control or select the antimigration force exerted by the anti-migration component **120, 220, 320, 420, 520, 620** on the associated respective tissue-engaging component **110, 210, 310, 410, 510, 610.** If the inflatable anti-migration component **120, 220, 320, 420, 520, 620** is a balloon formed of a non-compliant material, the balloon may be sized, shaped, configured, and/or dimensioned so that it exerts only up to a preselected expansion force.

In some embodiments, such as illustrated in in **FIG. 2****,** the radially-outwardly directed force of the anti-migration component **220** of an occlusion device **200** may be sufficient against the tissue-engaging component **210** (e.g., within the lumen **215** of the saddle region **216)** to hold the anti-migration component **220** in place with respect to the tissue-engaging component **210.** However, in addition to inhibiting or preventing migration of a tissue-engaging component, in some embodiments, the anti-migration component may also include features configured to inhibit or prevent migration of the anti-migration component itself with respect to the associated tissue-engaging component and/or the deployment site. Various examples of anti-migration components **320, 420, 520, 620** of respective examples of embodiments of occlusion devices **300, 400, 500, 600** illustrated **FIG. 3****,** **FIG. 4****,** **FIG. 5****.** and **FIG. 6****,** respectively, are configured to inhibit or prevent migration with respect to the associated tissue-engaging component **310, 410, 510, 610** and/or the deployment site. For instance, at least one end of the anti-migration component may be expanded to resist migration from the lumen of the saddle region of the tissue-engaging component and/or from a body passage through which the occlusion device is deployed.

In the example of an embodiment of an occlusion device **300** illustrated in **FIG. 3****,** an anti-migration component **320** may be configured to inhibit or prevent migration thereof with respect to the tissue-engaging component **310.** More particularly, the anti-migration component **320** of the occlusion device **300** illustrated in **FIG. 3** has a distal retention members **322** extending from a distal end **321** of the anti-migration component **320,** and a proximal retention member **324** extending from a proximal end **323** of the anti-migration component **320.** The diameters of the retention members **322, 324** (in a direction transverse to the longitudinal axis **LA** of the occlusion device **300)** are larger than the diameter of the lumen **315** through the saddle region **316** of the tissue-engaging component **310** of the occlusion device **300** (in a direction transverse to the longitudinal axis **LA** of the occlusion device **300)** so that the anti-migration component **320** is inhibited or prevented from slipping through the lumen **315** and migrating with respect to the tissue-engaging component **310.** The anti-migration component **320** may be an expandable balloon, formed of a non-compliant material pre-shaped so that the anti-migration component **320** expands to a "dogbone" shape as illustrated in **FIG. 3****.** However, other configurations are within the scope and spirit of the present disclosure, the specific shape not being limited to dogbone.

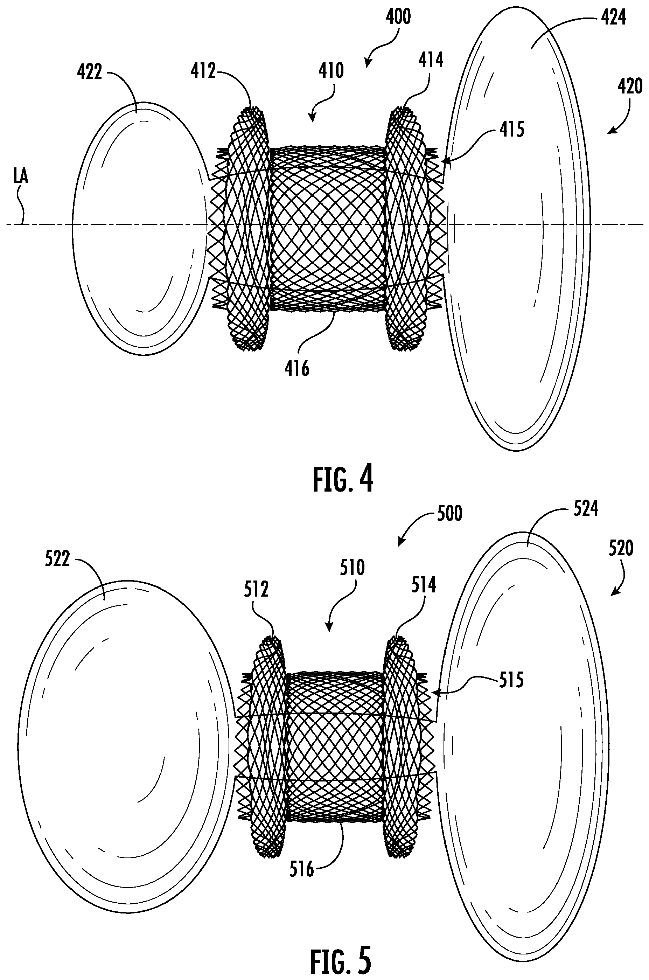

In addition to being configured to inhibit or prevent migration with respect to the tissue-engaging component, an anti-migration component formed in accordance with various principles of the present disclosure may also be configured to inhibit or prevent migration with respect to the deployment site. For instance, an anti-migration component of an occlusion device positioned across a body passage may be configured with antimigration features which inhibit or prevent migration of the anti-migration component with respect to the body passage. In some embodiments, the anti-migration component may include retention members, such as expanded members, at one or both ends thereof, sized, shaped, configured, and/or dimensioned to be seated (e.g., anchored) against tissue surrounding the body passage in which the implantable device is positioned to hold the anti-migration component in place with respect to the body passage. Examples of embodiments of occlusion devices **400, 500, 700** having an anti-migration component with at least one retention member sized, shaped, configured, and/or dimensioned to not only retain the anti-migration component with respect to the tissue-engaging component but also to retain the anti-migration component in place with respect to the deployment site are illustrated in **FIG. 4, FIG. 5****,** **FIG. 6,** and **FIG. 7****.**

In the example of an embodiment of an occlusion device **400** illustrated in **FIG. 4****,** an anti-migration component **420** has a proximal retention member **424** sized, shaped, configured, and/or dimensioned to inhibit or prevent migration from the lumen **415** defined through the saddle region **416** of the tissue-engaging component **410** (in which the anti-migration component **420** is positioned) as well as to inhibit or prevent migration from the deployment site. For instance, in the example of an embodiment illustrated in **FIG. 4****,** the proximal retention member **424** may be enlarged to have a diameter (in a direction transverse to the longitudinal axis **LA** of the occlusion device **400)** larger than the diameter of the lumen **415** defined through the saddle region **416** of the tissue-engaging component **410** of the occlusion device **400,** as well as larger than the body passage through which the saddle region **416** is configured to be positioned, and configured to resist migrating through the lumen **415** as well as the body passage. The illustrated configuration may be suitable for placement of the occlusion device **400** across a pylorus **P,** such as in **FIG. 1****,** with the proximal retention member **424** positioned within the stomach **S** and having a diameter wider than the diameter of the pylorus **P** to resist migration distally through the pylorus **P** and into the duodenum **D.** The distal retention member **422** may be smaller than the proximal retention member **424,** as illustrated in **FIG. 4****,** or the same size or larger than the proximal retention member **424,** such as generally depending on the characteristics of the deployment site. As may be appreciated, the proximal retention member **424** may also wedge the proximal retention member **414** of the tissue-engaging component **410** in place with respect to the deployment site to inhibit or prevent migration of the tissue-engaging component **410** in such manner as well.

In the example of an embodiment of an occlusion device **500** illustrated in **FIG. 5****,** both the distal retention member **522** as well as the proximal retention member **524** of the anti-migration component **520** are configured to inhibit or prevent migration through the lumen **515** defined through the saddle region **516** of the tissue-engaging component **510** (in which the anti-migration component **520** is positioned) as well as to inhibit or prevent migration from the deployment site. For instance, in the example of an embodiment illustrated in **FIG. 5** both the proximal retention member **524** as well as the distal retention member **522** may be enlarged to have a diameter (in a direction transverse to the longitudinal axis **LA** of the occlusion device **500)** larger than the diameter of the lumen **515** defined through the saddle region **516** of the tissue-engaging component **510** of the occlusion device **500,** as well as larger than the body passage through which the saddle region **516** is configured to be positioned, and configured to resist migrating through the lumen **515** as well as the body passage. The illustrated configuration may be suitable for placement of the occlusion device **500** across a pylorus **P,** such as in **FIG. 1****,** with the proximal retention member **524** positioned within the stomach **S,** and with the distal retention member **522** positioned within the duodenum **D,** and with both the proximal retention member **524** and the distal retention member **522** having a diameter wider than the diameter of the pylorus **P** to resist migration of the anti-migration component **520** distally or proximally through the pylorus **P** and into the duodenum **D** or stomach **S.** As may be appreciated, the proximal retention member **524** of the anti-migration component **520** may also wedge the proximal retention member **514** of the tissue-engaging component **510** in place with respect to the deployment site, and the distal retention member **522** may also wedge the distal retention member **512** of the tissue-engaging component **510** in place with respect to the deployment site to inhibit or prevent migration of the tissue-engaging component **510** in such manner as well.

In accordance with various principles of the present disclosure, at least one of the retention members of the occlusion device is sized, shaped, configured, and dimensioned to correspond to the anatomy in which such retention member is to be seated. For instance, in the example of an embodiment of an occlusion device **600** illustrated in **FIG. 6****,** the distal retention member **622** of the anti-migration component **620** is sized, shaped, configured, and dimensioned to correspond to the anatomical contours of the duodenum **D,** as illustrated in **FIG. 7****.** More particularly, the distal retention member **622** may be curved or bent or otherwise not extend completely along the longitudinal axis **LA** of the occlusion device **600,** such as to correspond with a typical bend in the duodenum **D** at the distal opening of the pylorus **P** into the duodenum **D.** Similar to the proximal retention members of the above-described embodiments, the proximal retention member **624** is sized, shaped, configured, and/or dimensioned to correspond to the anatomical contours of the stomach **S.**

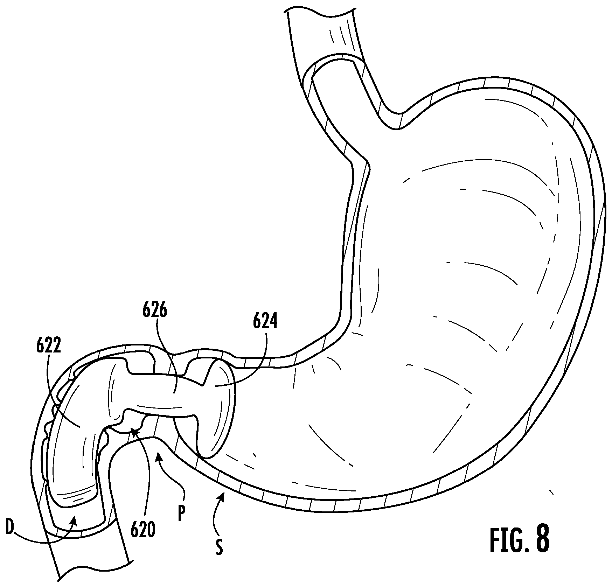

In some embodiments, an anti-migration component **620** of an occlusion device **600** such as illustrated in **FIG. 6** and **FIG. 7** may be sized, shaped, configured, and/or dimensioned to be retained within an anatomical structure such as a pylorus **P** (and stomach **S** and duodenum **D)** on its own, without the tissue-engaging component **610,** such as illustrated in **FIG. 8****.** The anti-migration component **620** (used with or without a tissue-engaging component **610)** may be an expandable element, such as a balloon, which is expanded into a predetermined size, shape, and/or configuration to conform with at least a portion of the anatomical structure of the deployment site as well as to create at least one retention member sufficiently large enough to anchor the anti-migration component in place. For instance, as illustrated in **FIG. 8****,** the anti-migration component **620** has a distal retention member **622** sized, shaped, configured, and/or dimensioned to substantially conform with the shape of a duodenum **D** in which the distal retention member **622** is deployed, and a saddle region **626** sized, shaped, configured, and/or dimensioned to extend across a pylorus **P.** The extended and bent configuration of the distal retention member **622** may enhance retention of the distal retention member **622** within the duodenum **D.** The proximal retention member **624** may be enlarged, as in the previously-described occlusion devices, to resist distal migration from the stomach S towards the duodenum **D.** The anti-migration component **620** may be formed from a semi-compliant material which is formed or shaped to determine the ultimate size, shape, configuration, and/or dimensions to which the anti-migration component **620** expands. The saddle region **226** may be sized, shaped, configured, and/or dimensioned to engage the walls of the anatomical structure in which it is placed (e.g., a pylorus **P)** with sufficient force to resist migration of the anti-migration component **620** with respect to the anatomical structure. In the case of a pylorus **P** (or other anatomical structure which may not have a constant or fixed shape and/or diameter), the saddle region **226** may be sized, shaped, configured, and/or dimensioned to exert anti-migration force against the pylorus **P,** yet, if formed of a semi-compliant material, may allow for a degree of compression in response to contractions of the pylorus **P** (or reductions in diameter or other shifting of another anatomical structure).

As noted above, an anti-migration component **120, 220, 320, 420, 520, 620** formed in accordance with various principles of the present disclosure may be a balloon formed of a compliant or non-compliant material preformed (e.g., molded) into a shape configured to resist or prevent migration through a body passage through which the anti-migration component **120, 220, 320, 420, 520, 620** is positioned. Such anti-migration component **120, 220, 320, 420, 520, 620** may also occlude passage of materials through a lumen **115, 215, 315, 415, 515*,* 615** of a tissue-engaging component **110, 210 310, 410, 510, 610** through which the anti-migration component **120, 220, 320, 420, 520, 620** is positioned. As such, the anti-migration component **120, 220, 320, 420, 520, 620** may also be considered an occlusion component of the occlusion device **100, 200, 300, 400, 500, 600.** It will be appreciated that the anti-migration component **120, 220, 320, 420, 520, 620** may be used independently of a separate tissue-engaging component **110, 210 310, 410, 510, 610,** thereby functioning as the tissue-engaging component as well as the anchoring anti-migration component of the occlusion device **100, 200, 300, 400, 500, 600.** In such embodiment of an occlusion device, the retention members as well as the intermediate saddle region extending between the proximal and distal ends of the anti-migration component could serve as anti-migration components as well as tissue-engaging components of the occlusion device.

To remove the occlusion device **100, 200, 300, 400, 500, 600,** such as after a defined term *in situ,* expandable components thereof are reduced in size (e.g., contracted, compressed, etc.) for removal, preferably transluminally or in an otherwise non-invasive manner (e.g., via a catheter or other means known or heretofore developed in the art). In an embodiment in which the tissue-engaging component **110, 210, 310, 410, 510, 610** is a stent, such stent is caused to collapse or otherwise return to a compact configuration (in accordance with a variety of manners known in the art) for transluminal removal from the body. For instance, a removal element **130,** such as illustrated in **FIG. 1****,** may be operatively associated with the tissue-engaging component **110** to facilitate shifting of the tissue-engaging component **110** into a compact configuration in a manner known in the art. The removal element **130** preferably is large enough to be readily grasped by a device inserted in a minimally invasive manner, such as endoscopically or transluminally, and pulled to return the tissue-engaging component **110** from its expanded deployed configuration to a collapsed delivery configuration suitable for removal in a minimally invasive manner, such as endoscopically or transluminally. In an embodiment in which the anti-migration component **120, 220, 320, 420, 520, 620** is a balloon, such component is deflated (in accordance with any of a variety of manners known in the art) for transluminal removal from the body. For instance, the filling fluid within the balloon may be released therefrom to cause the balloon to return to a sufficiently unexpanded configuration for removal from the body. In some embodiments, the balloon includes an inflation valve which may be actuated (e.g., removed, opened, broken, or otherwise disengaged) to release the fluid from the interior of the balloon. Additionally or alternatively, the balloon integrity can be disrupted to release the fluid therein, such as by being punctured. A needle, such as for FNA (fine needle aspiration), may be used to aspirate fluid or evacuate gas from the balloon, either through the balloon wall or through an inflation valve used to inflate the balloon. The unexpanded or deflated balloon may be grasped or otherwise retrieved and removed in any desired manner known in the art or heretofore developed.

It will be appreciated that various aspects of the present disclosure may be applied to occlusion devices configured for placement in other passages within the body to reduce flow through such passage. Moreover, it will be appreciated that various aspects of the present disclosure may be applied to deployment sites other than body passages.

Various structures and features of the embodiments described herein and illustrated in the figures have several separate and independent unique benefits which may be desirable for, yet not critical to, the presently disclosed occlusion device. Therefore, the various structures and features described herein need not all be present in order to achieve at least some of the desired characteristics and/or benefits described herein. Moreover, the various features described herein may be used singly or in any combination. It will be appreciated that various features described with respect to one embodiment may be applied to another embodiment, whether or not explicitly indicated. Thus, it should be understood that one or more of the features described with reference to one embodiment can be combined with one or more of the features of any of the other embodiments described herein. That is, any of the features described herein can be mixed and matched to create hybrid designs, and such hybrid designs are within the scope of the present disclosure. Therefore, the present invention is not limited to only the embodiments specifically described herein.

## Claims

1. An implantable device comprising:
a tissue-contacting component (110, 210, 310, 410, 510, 610) configured to resist tissue ingrowth; and
an anti-migration component (120, 220, 320, 420, 520, 620) separately formed from said tissue-contacting component (110, 210, 310, 410, 510, 610) and configured to hold said tissue-contacting component (110, 210, 310, 410, 510, 610) in place with respect to tissue at a deployment site, wherein the anti-migration component (120, 220, 320, 420, 520, 620) is an inflatable balloon and **characterised in that** the anti-migration component is positioned witnin a lumen or the tissue-contacting component (110, 210, 310, 410, 510, 610).

2. The implantable device of claim 1, wherein said tissue-contacting component (110, 210, 310, 410, 510, 610) is coated with a material resistant to tissue ingrowth.

3. The implantable device of any of claims 1-2, wherein at least one of said tissue-contacting component (110, 210, 310, 410, 510, 610) or said anti-migration component (120, 220, 320, 420, 520, 620) is expandable from a compact delivery configuration to an expanded deployment configuration.

4. The implantable device of any of claims 1-3, wherein:
said anti-migration component (120, 220, 320, 420, 520, 620) is expandable within the lumen (115, 215, 315, 415, 515, 615) defined through said tissue-contacting component (110, 210, 310, 410, 510, 610) to exert an anti-migration force on said tissue-contacting component (110, 210, 310, 410, 510, 610) to hold said tissue-contacting component (110, 210, 310, 410, 510, 610) in place with respect to tissue at the deployment site.

5. The implantable device of any of claims 1-4, wherein:
said tissue-contacting component (110, 210, 310, 410, 510, 610) is an expandable stent.

6. The implantable device of any of claims 1-3, wherein at least one of said tissue-contacting component (110, 210, 310, 410, 510, 610) or said anti-migration component (120, 220, 320, 420, 520, 620) includes at least one retention member (112, 114, 212, 214, 312, 314, 412, 414, 512, 514, 612, 614) extending radially-outwardly therefrom.

7. The implantable device of claim 6, wherein said at least one retention member (112, 114, 212, 214, 312, 314, 412, 414, 512, 514, 612, 614) extends radially outwardly from an end of said anti-migration component (120, 220, 320, 420, 520, 620).

8. The implantable device of any of claims 1-4, wherein:
said tissue-contacting component (110, 210, 310, 410, 510, 610) has a proximal end configured to be seated in a stomach, a distal end configured to be seated in a duodenum, and a saddle region (116, 216, 226, 316, 416, 516, 616, 626) extending between the proximal end and the distal end and configured to be seated in a pylorus; and
said anti-migration component (120, 220, 320, 420, 520, 620) has a proximal end with a proximal retention member (114, 214, 314, 414, 514, 614) extending radially therefrom and configured to be seated in the stomach, a distal end with a distal retention member (112, 212, 312, 412, 512, 612) extending radially therefrom and configured to be seated in the duodenum, and a saddle region (116, 216, 226, 316, 416, 516, 616, 626) extending between the proximal end and the distal end and configured to extend through the saddle region (116, 216, 226, 316, 416, 516, 616, 626) of said tissue-contacting component (110, 210, 310, 410, 510, 610).

9. The implantable device of claim 8, wherein said distal retention member (112, 212, 312, 412, 512, 612) is curved to correspond to the curve of the duodenum in which said distal retention member (112, 212, 312, 412, 512, 612) is seated.

10. The implantable device of any of claims 1-9, wherein the implantable device forms an occlusion device (100, 200, 300, 400, 500, 600, 700) configured to occlude a body passage.

11. The implantable device (100, 200, 300, 400, 500, 600, 700) of claim 10, wherein the anti-migration component occludes flow of materials through the lumen (115, 215, 315, 415, 515, 615) defined through said tissue-contacting component .

12. The implantable device (100, 200, 300, 400, 500, 600, 700) of claim 10 or 11 in combination with claim 7, wherein said at least one retention member (112, 114, 212, 214, 312, 314, 412, 414, 512, 514, 612, 614) has a diameter greater than the diameter of the lumen (115, 215, 315, 415, 515, 615) through said tissue-contacting component and is configured to resist migration through said tissue-contacting component.

13. The implantable device (100, 200, 300, 400, 500, 600, 700) of claim 12, wherein the diameter of said at least one retention member (112, 114, 212, 214, 312, 314, 412, 414, 512, 514, 612, 614) extending radially outwardly from an end of said anti-migration component is greater than the diameter of the body passage through which said tissue-contacting component extends and is configured to resist migration of said tissue-contacting component and said anti-migration component through the body passage.

## Patentansprüche

1. Implantierbare Vorrichtung, aufweisend:
eine gewebeberührende Komponente (110, 210, 310, 410, 510, 610), die zum Hemmen von Gewebeeinwuchs konfiguriert ist; und
eine Anti-Migrationskomponente (120, 220, 320, 420, 520, 620), die getrennt von der gewebeberührenden Komponente (110, 210, 310, 410, 510, 610) ausgebildet und zum Festhalten der gewebeberührenden Komponente (110, 210, 310, 410, 510, 610) in Bezug auf das Gewebe an einer Einsatzstelle konfiguriert ist, wobei die Anti-Migrationskomponente (120, 220, 320, 420, 520, 620) ein aufblasbarer Ballon ist, und
**dadurch gekennzeichnet ist, dass**
die Anti-Migrationskomponente in einem Lumen der gewebeberührenden Komponente (110, 210, 310, 410, 510, 610) angeordnet ist.

2. Implantierbare Vorrichtung nach Anspruch 1, wobei die gewebeberührende Komponente (110, 210, 310, 410, 510, 610) mit einem Material beschichtet ist, das Gewebeeinwuchs hemmt.

3. Implantierbare Vorrichtung nach einem der Ansprüche 1 bis 2, wobei mindestens die gewebeberührende Komponente (110, 210, 310, 410, 510, 610) oder die Anti-Migrationskomponente (120, 220, 320, 420, 520, 620) von einer kompakten Einführkonfiguration in eine expandierte Einsatzkonfiguration expandierbar ist.

4. Implantierbare Vorrichtung nach einem der Ansprüche 1 bis 3, wobei:
die Anti-Migrationskomponente (120, 220, 320, 420, 520, 620) innerhalb des durch die gewebeberührende Komponente (110, 210, 310, 410, 510, 610) definierten Lumens (115, 215, 315, 415, 515, 615) expandierbar ist, um eine Anti-Migrationskraft auf die gewebeberührende Komponente (110, 210, 310, 410, 510, 610) auszuüben, um die gewebeberührende Komponente (110, 210, 310, 410, 510, 610) in Bezug auf das Gewebe an der Einsatzstelle festzuhalten.

5. Implantierbare Vorrichtung nach einem der Ansprüche 1 bis 4, wobei:
die gewebeberührende Komponente (110, 210, 310, 410, 510, 610) ein expandierbarer Stent ist.

6. Implantierbare Vorrichtung nach einem der Ansprüche 1 bis 3, wobei mindestens die gewebeberührende Komponente (110, 210, 310, 410, 510, 610) oder die Anti-Migrationskomponente (120, 220, 320, 420, 520, 620) mindestens ein Rückhalteelement (112, 114, 212, 214, 312, 314, 412, 414, 512, 514, 612, 614) aufweist, das sich von dieser radial nach außen erstreckt.

7. Implantierbare Vorrichtung nach Anspruch 6, wobei sich das mindestens eine Rückhalteelement (112, 114, 212, 214, 312, 314, 412, 414, 512, 514, 612, 614) von einem Ende der Anti-Migrationskomponente (120, 220, 320, 420, 520, 620) radial nach außen erstreckt.

8. Implantierbare Vorrichtung nach einem der Ansprüche 1 bis 4, wobei:
die gewebeberührende Komponente (110, 210, 310, 410, 510, 610) ein proximales Ende, das dazu konfiguriert ist, in einem Magen zu sitzen, ein distales Ende, das dazu konfiguriert ist, in einem Zwölffingerdarm zu sitzen, und einen Sattelbereich (116, 216, 226, 316, 416, 516, 616, 626) aufweist, der sich zwischen dem proximalen Ende und dem distalen Ende erstreckt und dazu konfiguriert ist, in einem Magenausgang zu sitzen; und
die Anti-Migrationskomponente (120, 220, 320, 420, 520, 620) ein proximales Ende mit einem proximalen Rückhalteelement (114, 214, 314, 414, 514, 614) aufweist, das sich radial von diesem aus erstreckt und dazu konfiguriert ist, im Magen zu sitzen, ein distales Ende mit einem distalen Rückhalteelement (112, 212, 312, 412, 512, 612), das sich radial von diesem aus erstreckt und dazu konfiguriert ist, im Zwölffingerdarm zu sitzen, und einen Sattelbereich (116, 216, 226, 316, 416, 516, 616, 626), der sich zwischen dem proximalen Ende und dem distalen Ende erstreckt und dazu konfiguriert ist, sich durch den Sattelbereich (116, 216, 226, 316, 416, 516, 616, 626) der gewebeberührenden Komponente (110, 210, 310, 410, 510, 610) zu erstrecken.

9. Implantierbare Vorrichtung nach Anspruch 8, wobei das distale Rückhalteelement (112, 212, 312, 412, 512, 612) entsprechend der Krümmung des Zwölffingerdarms gekrümmt ist, in dem das distale Rückhalteelement (112, 212, 312, 412, 512, 612) sitzt.

10. Implantierbare Vorrichtung nach einem der Ansprüche 1 bis 9, wobei die implantierbare Vorrichtung eine Verschlussvorrichtung (100, 200, 300, 400, 500, 600, 700) bildet, die zum Verschließen eines Körperdurchgangs konfiguriert ist.

11. Implantierbare Vorrichtung (100, 200, 300, 400, 500, 600, 700) nach Anspruch 10, wobei die Anti-Migrationskomponente den Strom von Materialien durch das durch die gewebeberührende Komponente definierte Lumen (115, 215, 315, 415, 515, 615) blockiert.

12. Implantierbare Vorrichtung (100, 200, 300, 400, 500, 600, 700) nach Anspruch 10 oder 11 in Kombination mit Anspruch 7, wobei das mindestens eine Rückhalteelement (112, 114, 212, 214, 312, 314, 412, 414, 512, 514, 612, 614) einen Durchmesser aufweist, der größer als der Durchmesser des Lumens (115, 215, 315, 415, 515, 615) durch die gewebeberührende Komponente und dazu konfiguriert ist, eine Bewegung durch die gewebeberührende Komponente zu hemmen.

13. Implantierbare Vorrichtung (100, 200, 300, 400, 500, 600, 700) nach Anspruch 12, wobei der Durchmesser des mindestens einen Halteelements (112, 114, 212, 214, 312, 314, 412, 414, 512, 514, 612, 614), das sich von einem Ende der Anti-Migrationskomponente radial nach außen erstreckt, größer als der Durchmesser des Körperdurchgangs ist, durch den sich die gewebeberührende Komponente erstreckt, und dazu konfiguriert ist, eine Bewegung der gewebeberührenden Komponente und der Anti-Migrationskomponente durch den Körperdurchgang zu hemmen.

## Revendications

1. Dispositif implantable comprenant :
un composant de contact avec le tissu (110, 210, 310, 410, 510, 610) configuré pour résister à la croissance du tissu ; et
un composant anti-migration (120, 220, 320, 420, 520, 620) formé séparément dudit composant de contact avec le tissu (110, 210, 310, 410, 510, 610) et configuré pour maintenir ledit composant de contact avec le tissu (110, 210, 310, 410, 510, 610) en place par rapport au tissu sur un site de déploiement, dans lequel le composant anti-migration (120, 220, 320, 420, 520, 620) est un ballonnet gonflable et **caractérisé en ce que**
le composant anti-migration est positionné à l'intérieur d'une lumière du composant de contact avec le tissu (110, 210, 310, 410, 510, 610).

2. Dispositif implantable selon la revendication 1, dans lequel ledit composant de contact avec le tissu (110, 210, 310, 410, 510, 610) est recouvert avec un matériau résistant à la croissance du tissu.

3. Dispositif implantable selon l'une quelconque des revendications 1 à 2, dans lequel au moins l'un parmi ledit composant de contact avec le tissu (110, 210, 310, 410, 510, 610) ou ledit composant anti-migration (120, 220, 320, 420, 520, 620) est expansible à partir d'une configuration de pose compacte jusqu'à une configuration de déploiement expansée.

4. Dispositif implantable selon l'une quelconque des revendications 1 à 3, dans lequel :
ledit composant anti-migration (120, 220, 320, 420, 520, 620) est expansible à l'intérieur de la lumière (115, 215, 315, 415, 515, 615) définie à travers ledit composant de contact avec le tissu (110, 210, 310, 410, 510, 610) pour exercer une force anti-migration sur ledit composant de contact avec le tissu (110, 210, 310, 410, 510, 610) afin de maintenir ledit composant de contact avec le tissu (110, 210, 310, 410, 510, 610) en place par rapport au tissu sur le site de déploiement.

5. Dispositif implantable selon l'une quelconque des revendications 1 à 4, dans lequel :
ledit composant de contact avec le tissu (110, 210, 310, 410, 510, 610) est un stent expansible.

6. Dispositif implantable selon l'une quelconque des revendications 1 à 3, dans lequel au moins l'un parmi ledit composant de contact avec le tissu (110, 210, 310, 410, 510, 610) ou ledit composant anti-migration (120, 220, 320, 420, 520, 620) comprend au moins un élément de retenue (112, 114, 212, 214, 312, 314, 412, 414, 512, 514, 612, 614) s'étendant radialement vers l'extérieur à partir de ce dernier.

7. Dispositif implantable selon la revendication 6, dans lequel ledit au moins un élément de retenue (112, 114, 212, 214, 312, 314, 412, 414, 512, 514, 612, 614) s'étend radialement vers l'extérieur à partir d'une extrémité dudit composant anti-migration (120, 220, 320, 420, 520, 620).

8. Dispositif implantable selon l'une quelconque des revendications 1 à 4, dans lequel :
ledit composant de contact avec le tissu (110, 210, 310, 410, 510, 610) a une extrémité proximale configurée pour être installée dans un estomac, une extrémité distale configurée pour être installée dans un duodénum et une région de selle (116, 216, 226, 316, 416, 516, 616, 626) s'étendant entre l'extrémité proximale et l'extrémité distale et configurée pour être installée dans un pylore ; et
ledit composant anti-migration (120, 220, 320, 420, 520, 620) a une extrémité proximale avec un élément de retenue proximal (114, 214, 314, 414, 514, 614) s'étendant radialement à partir cette dernière et configurée pour être installée dans l'estomac, une extrémité distale avec un élément de retenue distal (112, 212, 312, 412, 512, 612) s'étendant radialement à partir de cette dernière et configurée pour être installée dans le duodénum, et une région de selle (116, 216, 226, 316, 416, 516, 616, 626) s'étendant entre l'extrémité proximale et l'extrémité distale et configurée pour s'étendre à travers la région de selle (116, 216, 226, 316, 416, 516, 616, 626) dudit composant de contact avec le tissu (110, 210, 310, 410, 510, 610).

9. Dispositif implantable selon la revendication 8, dans lequel ledit élément de retenue distal (112, 212, 312, 412, 512, 612) est courbé pour correspondre à la courbe du duodénum dans lequel ledit élément de retenue distal (112, 212, 312, 412, 512, 612) est installé.

10. Dispositif implantable selon l'une quelconque des revendications 1 à 9, dans lequel le dispositif implantable forme un dispositif d'occlusion (100, 200, 300, 400, 500, 600, 700) configuré pour boucher un passage corporel.

11. Dispositif implantable (100, 200, 300, 400, 500, 600, 700) selon la revendication 10, dans lequel le composant anti-migration empêche l'écoulement de matériaux à travers la lumière (115, 215, 315, 415, 515, 615) définie par ledit composant de contact avec le tissu.

12. Dispositif implantable (100, 200, 300, 400, 500, 600, 700) selon la revendication 10 ou 11 en combinaison avec la revendication 7, dans lequel ledit au moins un élément de retenue (112, 114, 212, 214, 312, 314, 412, 414, 512, 514, 612, 614) a un diamètre supérieur au diamètre de la lumière (115, 215, 315, 415, 515, 615) à travers ledit composant de contact avec le tissu et est configuré pour résister à la migration à travers ledit composant de contact avec le tissu.

13. Dispositif implantable (100, 200, 300, 400, 500, 600, 700) selon la revendication 12, dans lequel le diamètre dudit au moins un élément de retenue (112, 114, 212, 214, 312, 314, 412, 414, 512, 514, 612, 614) s'étendant radialement vers l'extérieur à partir d'une extrémité dudit composant anti-migration est supérieur au diamètre du passage corporel à travers lequel ledit composant de contact avec le tissu s'étend et est configuré pour résister à la migration dudit composant de contact avec le tissu et ledit composant anti-migration à travers le passage corporel.
